# EUROPEAN PATENT SPECIFICATION

(11) **EP 2 059 543 B1**
(45) Date of publication and mention of the grant of the patent: **14.03.2012**
(21) Application number: 07806981.2
(22) Date of filing: 04.09.2007
(51) Int. Cl.: C08F 212/32, C09K 11/06, H01L 51/50, H05B 33/14

(54) **ORGANIC ELECTROLUMINESCENCE ELEMENT AND USE THEREOF**
ORGANISCHES ELEKTROLUMINESZENZELEMENT UND VERWENDUNG
ÉLÉMENT ÉLECTROLUMINESCENT ORGANIQUE ET SON UTILISATION

(30) Priority: 05.09.2006 JP 2006240292
(43) Date of publication of application: 20.05.2009
(73) Proprietor: Showa Denko K.K., Tokyo 105-0012 (JP)
(72) Inventor: TAKAHASHI, Yoshiaki, Chiba 267-0056 (JP)
(74) Representative: Strehl, Peter
(86) International application number: PCT/JP2007/067549
(87) International publication number: WO 2008/029935

(56) References cited:
- EP-A- 1 424 350
- WO-A-2005/021678
- WO-A-2005/061562
- WO-A-2006/001150
- WO-A-2006/135076
- WO-A-2007/020881

## Description

### TECHNICAL FIELD

The present invention relates to an organic electroluminescence element, more specifically, to an organic electroluminescence element using a phosphorescent compound, and use thereof.

### BACKGROUND ART

In recent years, in order to expand the application of an organic electroluminescence element (also called as "organic EL element" in the present specification), material development focusing on a phosphorescent compound that exhibits high luminescence efficiency has been made intensively.

In order to expand the application of the organic EL element particularly to the field of display devices, the development of a material not only having high luminescence efficiency but also allowing the devices to keep stable operation is essential.

Japanese Patent Laid-Open Publication No. 2003-526876 (Patent Document 1) discloses that the luminescence efficiency of organic EL elements is substantially enhanced by using an organic iridium complex as a phosphorescent compound. The iridium complex is exemplified by tris(2-(2-pyridyl) phenyl) iridium and its derivatives. Patent Document 1 describes that the luminescent color of the iridium complex is changed by replacing the substituents of ligands having the aromatic structure by alkyl or aryl groups. Further, Japanese Patent Laid-Open Publication No. 2001-247859 (Patent Document 2) exemplifies various kinds of groups as the substituents of tris(2-(2-pyridyl) phenyl) iridium.

As a method for forming a luminescent layer of organic EL elements, there are used generally a vacuum deposition process of low-molecular-weight organic compounds and a coating process of a solution of high-molecular-weight compounds. The coating process is advantageous because the production cost of elements is low and elements with large area can be produced easily. Element fabrication technology using the coating process has been desired to be further improved.

Japanese Patent Laid-Open Publication No. 2003-342325 (Patent Document 3) discloses that the service life of organic EL elements prepared by a coating process can be extended by using a copolymer of a charge-transporting compound and an iridium complex as the high-molecular-weight compound.

Patent Document 1: Japanese Patent Laid-Open Publication No. 2003-526876

Patent Document 2: Japanese Patent Laid-Open Publication No. 2001-247859

Patent Document 3: Japanese Patent Laid-Open Publication No. 2003-342325

Document WO 2005/061562 discloses an organic electroluminescent element having a luminescent layer containing a phosphorescent iridium complex in a charge transporting non-conjugated polymer matrix, wherein the phosphorescent iridium complex has one tertiary butyl group on each of the 2-phenylpyridine ligands.

### DISCLOSURE OF THE INVENTION

Regarding the organic EL elements using the iridium complexes described in Patent Documents 1 and 2, the service life (evaluated by lowering in luminosity after a constant current is applied to the elements) of the elements prepared by coating a solution containing a high-molecular-weight compound and an iridium complex is extremely short as compared with that of the elements prepared by vacuum evaporation process of the same iridium complex.

This is possibly because an element prepared by coating process that has a difficulty in building up organic charge injection layers has a high charge injection barrier from electrodes to the iridium complex as compared with an element that is prepared by building up plural organic charge injection layers between a luminescent layer and an electrode by vacuum evaporation, and thus still heavier electrical load is considered to be imposed on the luminescent layer in the former element.

This high charge injection barrier from electrodes to the iridium complex is not basically reduced by using such a copolymer as is described in Patent Document 3.

The present invention has been carried out in view of these circumstances. It is an object of the present invention to provide an organic EL element having high luminescence efficiency and a long service life as well.

The present inventor has conducted intensive studies to address the above problems and have found that an organic EL element containing a phosphorescent compound with a specific structure in a luminescent layer has a low charge injection barrier from electrodes and exhibits a long service life, thus accomplishing the present invention. Namely, the present invention is summarized as follows.
[1] An organic electroluminescence element comprising:
   a substrate;
   a pair of electrodes formed on the substrate; and
   one or plural organic layers formed between the pair of the electrodes, said organic layer containing a luminescent layer,
   wherein the luminescent layer contains a phosphorescent compound represented by the following formula (1) and a charge-transporting non-conjugated polymer, 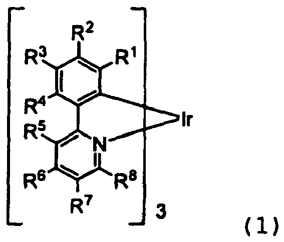
   in the formula (1), R¹ to R⁸ each are independently a hydrogen atom or an alkyl group having 1 to 20 carbon atoms; a plurality of the alkyl groups may be linked with one another to form a ring;
   at least two of R¹ to R⁸ are each the alkyl group; and
   at least one of the alkyl groups is an alkyl group having a tertiary or quaternary α-carbon atom.
[2] The organic electroluminescence element as described in [1], wherein all of the alkyl groups are each an alkyl group having a tertiary or quaternary α-carbon atom.
[3] The organic electroluminescence element as described in [2], wherein all of the alkyl groups having a tertiary or quaternary α-carbon atom are each a tertiary butyl group.
[4] An application equipped with an organic electroluminescence element as described in any one of [1] to [3], wherein the application is selected from a display device, a backlight, an electrophotography, an illumination light source, a recording light source, an exposure light source, a reading light source, a sign board, a display board, an interior accessory and an optical communication.

### EFFECT OF THE INVENTION

The aforementioned Patent Documents do not disclose that the service life of organic EL elements can be improved by appropriately selecting the kind, number, or position of substituents.

The present invention provides an organic EL element having a low charge injection barrier from electrodes, high luminescence efficiency, and long service life as well.

### BRIEF DESCRIPTION OF THE DRAWINGS

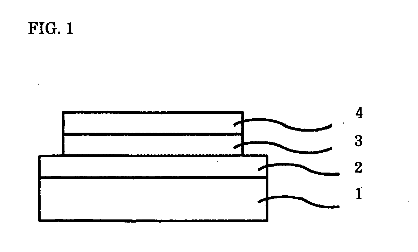

FIG. 1 is a schematic cross-sectional view illustrating an exemplary embodiment of an organic EL element according to the present invention.
   1 Glass substrate
   2 Anode
   3 Luminescent layer
   4 Cathode

### BEST MODE FOR CARRYING OUT THE INVENTION

The present invention will be described in detail below.

### 1. An embodiment in which a luminescent layer contains a luminescent low-molecular-weight compound

In the embodiment of the present invention, an organic EL element relating to the present invention comprises a substrate, a pair of electrodes formed on the substrate, and one or plural organic layers formed between the pair of the electrodes, said organic layer containing a luminescent layer, wherein the luminescent layer contains a phosphorescent compound represented by the following formula (1) and a charge-transporting non-conjugated polymer.

### <Phosphorescent compound>

In the present invention, a phosphorescent compound (iridium complex) represented by the following formula (1) is used.

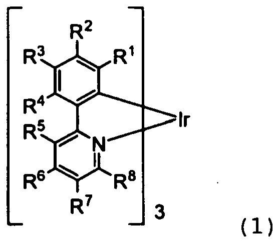

In the formula (1), R¹ to R⁸ each are independently hydrogen atom or an alkyl group having 1 to 20 carbon atoms; a plurality of the alkyl groups may be linked with one another to form a ring;
at least two of R¹ to R⁸ are each the alkyl group; and
at least one of the alkyl groups is an alkyl group having a tertiary or quaternary α-carbon atom (the "α-carbon atom" is a carbon atom that is contained in the alkyl group and adjacent to a carbon atom of the aromatic ring bonding to Ir (iridium atom)).

It is considered that the oxidation potential of the compound represented by the formula (1) is lower than that of tris(2-(2-pyridyl) phenyl) iridium that has been used so far, and therefore an organic EL element containing the compound of the formula (1) as a phosphor has a lower injection barrier from electrodes to the iridium complex, reducing electrical load imposed on the luminescent layer, and thereby attains an extended service life.

In addition to the lower oxidation potential, from the viewpoint of making the exited state of the reactive iridium complex be sterically-isolated from the other molecules contained in the luminescent layer and attaining an organic EL element with an extended service life, it is preferred that all of the alkyl groups in the formula (1) be each an alkyl group having a tertiary or quaternary α-carbon atom.

Further, in the formula (1), it is preferred that all of the alkyl groups having a tertiary or quaternary α-carbon atom be each a tertiary butyl group, because both of the effect of lowering the oxidation potential of the iridium complex and the effect of making the excited state of the reactive iridium complex be sterically-isolated from the other molecules contained in the luminescent layer are large, and because the organic EL elements advantageously attain extended service life. A plurality of the tertiary butyl groups may be linked with one another to form a ring as is shown by the complex of the following formula (C5).

A phosphorescent compound represented by the formula (1) in which any one of R¹ to R⁸ is an alkyl group having a tertiary or quaternary α-carbon atom has been known so far. However, a phosphorescent compound in which at least two of R¹ to R⁸ are each an alkyl group having a tertiary or quaternary α-carbon atom (particularly, a phosphorescent compound in which at least two of R¹ to R⁸ are each a tertiary butyl group) has not been specifically known so far. Further, it has not been known so far that an organic EL element using the latter phosphorescent compound has a particularly extended service life.

In the present invention, the phosphorescent compound may be used alone or in combination of two or more kinds.

As the compound represented by the formula (1), the complexes represented by the following formulae (C1) to (C6) are preferable, and the complex represented by the formula (C1) is particularly preferable, because an organic EL element having particularly extended service life and high luminescence efficiency is attained.

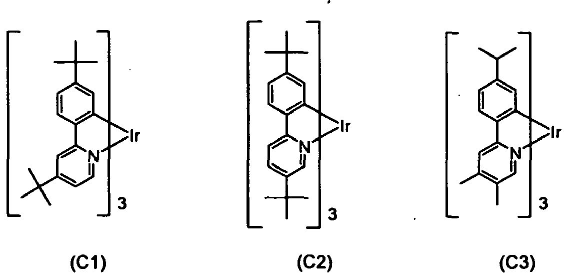

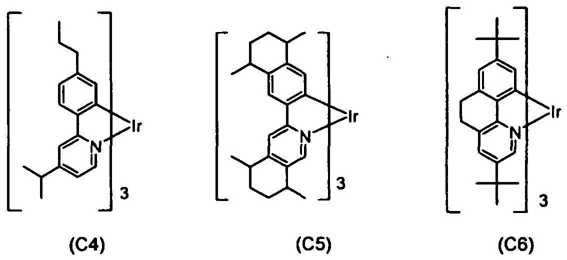

The iridium complex represented by the above formula (1) maybeproducedby, for example, a method comprising the following steps (i) and (ii) :
step (i) : reacting a compound represented by the following formula (1-1) with iridium (III) trichloride trihydrate in a solvent such as 2-ethoxyethanol at about 50 to 150°C to produce a compound represented by the following formula (1-2); and
step (ii): reacting the compound represented by the following formula (1-2) with the compound represented by the following formula (1-1) in the presence of a base such as potassium carbonate and in glycerin at about 150 to 220°C to produce the iridium complex represented by the above formula (1).

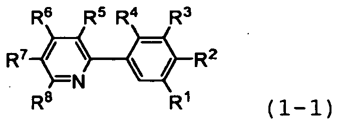

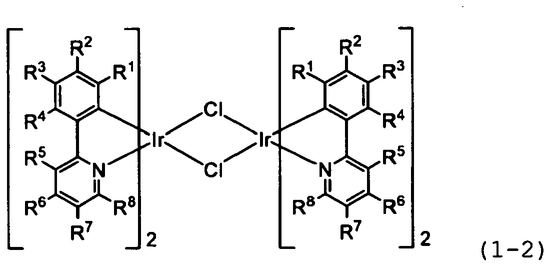

### <Charge-transporting non-conjugated polymer>

The charge-transporting non-conjugated polymer is preferably a polymer obtained by copolymerizing monomers containing at least one kind of polymerizable compound selected from the group consisting of a hole-transporting polymerizable compound and an electron-transporting polymerizable compound. In the present specification, the hole-transporting polymerizable compound and the electron-transporting polymerizable compound are also collectively called a charge-transporting polymerizable compound.

Namely, the charge-transporting non-conjugated polymer is preferably a polymer that contains a structural unit derived from one kind or two or more kinds of the hole-transporting polymerizable compounds or a structural unit derived from one kind or two or more kinds of the electron transporting polymerizable compounds. When such polymer is used, higher luminescence efficiency is obtained because a higher mobility of charges can be attained in the luminescent layer and a uniform thin film can be formed by coating.

Further, the charge-transporting non-conjugated polymer is more preferably a polymer that contains both a structural unit derived from one kind or two or more kinds of the hole-transporting polymerizable compounds and a structural unit derived from one kind or two or more kinds of the electron transporting polymerizable compounds. When such polymer is used, still higher luminescence efficiency is obtained because the polymer has both of hole-transporting and electron-transporting functions, and thus holes and electrons recombine more efficiently in the vicinity of the phosphorescent compound.

There is no particular limitation on the hole-transporting polymerizable compounds and electron-transporting polymerizable compounds as long as they have a polymerizable functional group or a substituent containing a polymerizable functional group, and known charge-transporting compounds may be used.

Examples of the polymerizable functional group include radical, cation, anion, addition, and
condensation-polymerizable functional groups. Among these, a radical polymerizable functional group is preferable because polymers can be easily produced.

Examples of the polymerizable functional group include allyl group, alkenyl group, acrylate group, methacrylate group, urethane (meth)acrylate group such as methacryloyloxy ethylcarbamate group and the like, vinylamide group, and their derivatives. Among these, alkenyl group is preferable.

More specifically, in the case where the polymerizable functional group is alkenyl group, the charge-transporting polymerizable compound preferably has a substituent represented by the following formulae (A1) to (A12) as the polymerizable functional group or the substituent containing a polymerizable functional group. Among, these, the substituents represented by the formulae (A1), (A5), (A8), and (A12) are more preferable, because these substituents can be easily introduced into the charge-transporting compounds.

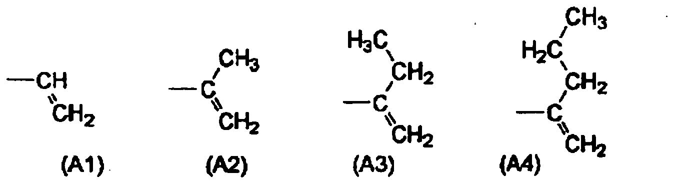

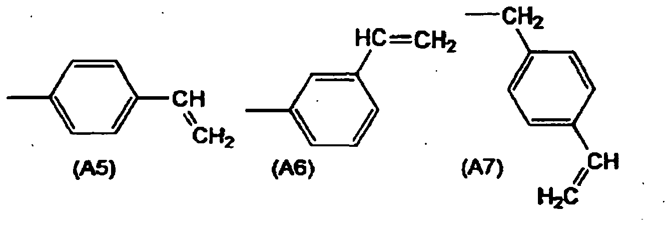

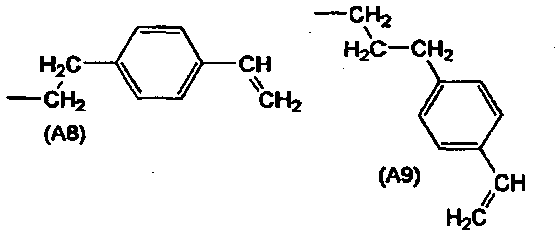

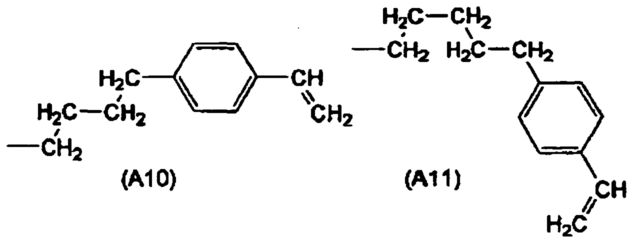

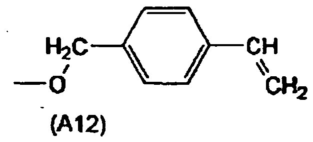

Specifically, the hole-transporting polymerizable compounds are preferably the compounds represented by the following formulae (E1) to (E6). From the viewpoint of charge mobility in the non-conjugated polymers, the compounds represented by the formulae (E1) to (E3) are more preferable.

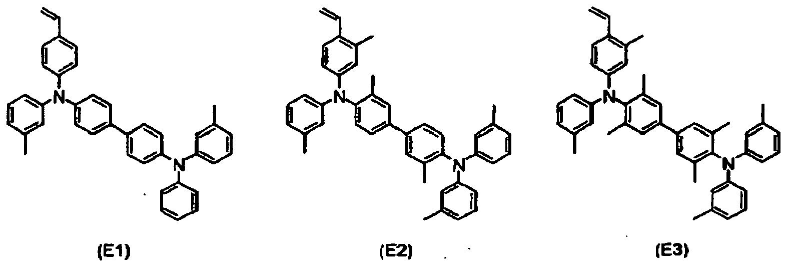

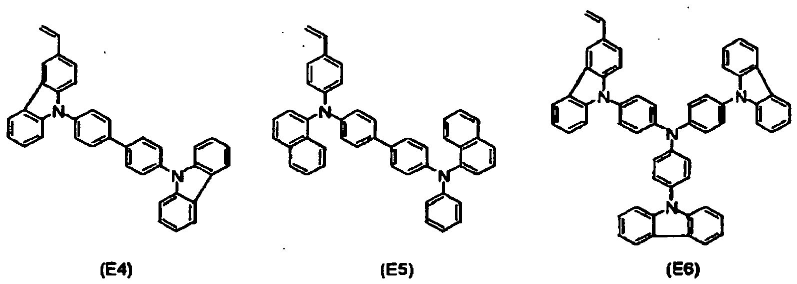

Specifically, the electron-transporting polymerizable compounds are preferably the compounds represented by the following formulae (E7) to (E15). From the viewpoint of charge mobility in the non-conjugated polymers, the compounds represented by the formulae (E7) and (E12) to (E14) are more preferable.

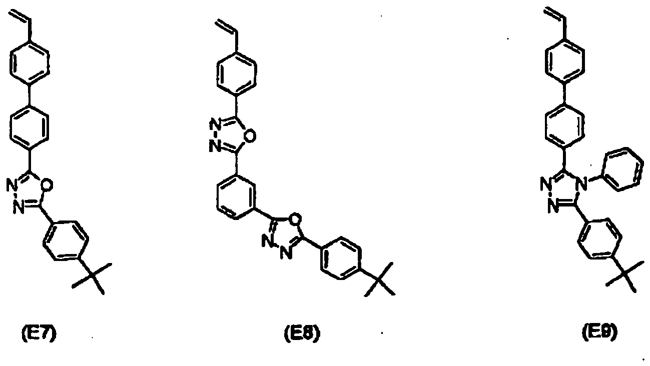

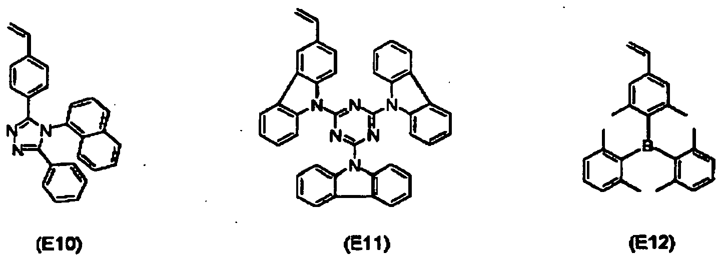

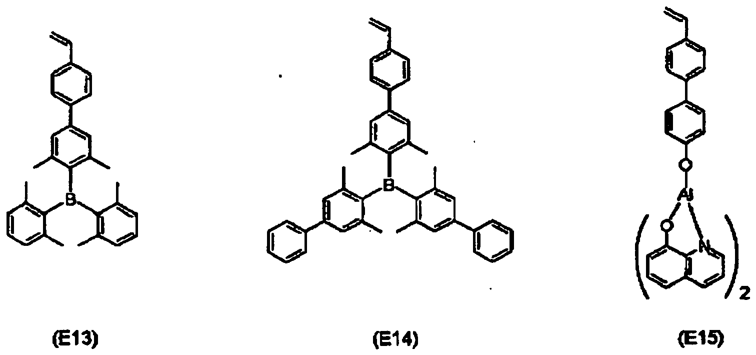

A compound given by replacing the substituent represented by the formula (A1) in the formulae (E1) to (E15) by a substituent represented by any of the formulae (A2) to (A12) may be preferably used. In particular, a compound having a substituent represented by the formulae (A1) or (A5) is preferable, because the functional group may be introduced easily to the polymerizable compounds.

As the charge-transporting non-conjugated polymer, more preferable are compounds given by copolymerizing a compound represented by any of the formulae (E1) to (E3) as the hole-transporting polymerizable compound, and a compound represented by any of the formulae (E7) and (E12) to (E14) as the electron-transporting polymerizable compound among these. When these non-conjugating polymers are used, holes and electrons recombine more efficiently on the phosphorescent compound and higher luminescence efficiency is obtained. In addition, in combination with the phosphorescent compound, these compounds can form an organic layer having a uniform distribution, providing an organic EL element having an excellent durability.

In the organic layer (luminescent layer) used for an organic EL element of the present invention and containing the phosphorescent compound and the non-conjugated polymer, the phosphorescent compound is contained in the matrix formed from the non-conjugated polymer in a dispersed state. As a result, luminescence that is not easy to use conventionally, namely the luminescence by way of the triplet excited state of the phosphorescent compound can be attained. Therefore, higher luminescence efficiency is obtained by using the organic layer.

The charge-transporting non-conjugated polymer may contain a structural unit derived from the other polymerizable compounds as long as the object of the present invention is not impaired. The other polymerizable compounds are, for example, compounds having no charge-transporting properties exemplified by alkyl(meth)acrylates such as methyl acrylate and methyl methacrylate, and styrene and its derivatives, but are not limited to them.

The charge-transporting non-conjugated polymers have a weight average molecular weight of preferably from 1,000 to 2,000,000, and more preferably from 5,000 to 1,000,000. In the present specification, the molecular weight represents the molecular weight with reference to polystyrene measured with GPC (gel permeation chromatography). It is preferable that the molecular weight is within the above range because the polymer is soluble in an organic solvent and uniform thin films are obtained.

The charge-transporting non-conjugated polymers may be any of random, block, and alternate copolymers.

The charge-transporting non-conjugated polymers may be prepared by any method of radical, cation, anion, and addition polymerization, but radical polymerization is preferred.

### 2. An embodiment in which a luminescent layer contains a luminescent polymer (not belonging to the scope of the present invention)

An organic EL element may comprise a substrate, a pair of electrodes formed on the substrate, and one or plural organic layers formed between the pair of the electrodes, said organic layer containing a luminescent layer, wherein the luminescent layer contains a non-conjugated polymer (hereinafter, called as "non-conjugated polymer (2)" in some cases) having a structural unit derived from a phosphorescent compound (iridium complex) represented by the following formula (2).

### <Structural unit derived from phosphorescent compound>

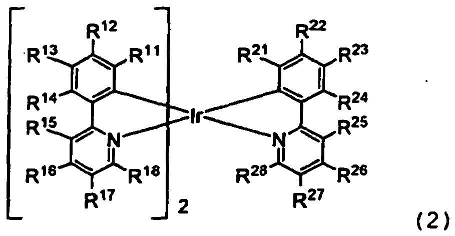

In the formula (2), R¹¹ to R¹⁸ each are independently hydrogen atom or an alkyl group having 1 to 20 carbon atoms; a plurality of the alkyl groups may be linked with one another to form a ring;
at least two of R¹¹ to R¹⁸ are each the alkyl group;
at least one of the alkyl groups is an alkyl group having a tertiary or quaternary α-carbon atom (the "α-carbon atom" is a carbon atom that is contained in the alkyl group and adjacent to a carbon atom of the aromatic ring bonding to Ir (iridium atom)).
R²¹ to R²⁸ each are independently selected from the group consisting of hydrogen atom, an alkyl group having 1 to 20 carbon atoms, and an alkenyl group having 2 to 20 carbon atoms; the hydrogen atom of the alkyl groups may be substituted with a polymerizable functional group; a plurality of the alkyl groups may be linked with one another to form a ring;
at least two of R²¹ to R²⁸ are not hydrogen atoms; and
at least one of R²¹ to R²⁸ is the alkyl group, at least one of whose hydrogen atoms is substituted with a polymerizable functional group, or the alkenyl group.

It is considered that the oxidation potential of the iridium complex represented by the formula (2) is lower than that of bis(2-(2-pyridyl)phenyl)(5-vinyl-2-(2-pyridyl)phenyl) iridium that has been used so far as a phosphorescent compound, wherein the substituents on its aromatic ring other than the substituent having a polymerizable functional group are hydrogen atoms, and therefore an organic EL element containing the non-conjugated polymer (2) as a phosphor has a lower injection barrier from electrodes to the iridium complex, reducing electrical load imposed on the luminescent layer, and thereby attains an extended service life.

In addition to the lower oxidation potential, from the viewpoint of making the exited state of the reactive iridium complex be sterically-isolated from the other molecules contained in the luminescent layer and attaining an organic EL element having an extended service life, it is preferred that at least two of R¹¹ to R¹⁸ are each a tertiary butyl group in the formula (2). A plurality of the tertiary butyl groups may be linked with one another to form a ring.

A phosphorescent compound represented by the formula (2) in which any one of R¹¹ to R¹⁸ is an alkyl group having a tertiary or quaternary α-carbon atom has been known so far. However, a phosphorescent compound in which at least two of R¹¹ to R¹⁸ are each an alkyl group having a tertiary or quaternary α-carbon atom (particularly, a phosphorescent compound in which at least two of R¹¹ to R¹⁸ are each a tertiary butyl group) has not been specifically known so far. Further, it has not been known so far that an organic EL element using the latter phosphorescent compound has a particularly extended service life.

Examples of the substituent having the polymerizable functional group includes any of radical, cation, anion, addition, and condensation-polymerizable functional group. Among these, a radical polymerizable functional group is preferable because polymers can be easily produced.

Examples of the polymerizable functional group include allyl group, alkenyl group, acrylate group, methacrylate group, urethane (meth)acrylate group such as methacryloyloxy ethylcarbamate group and the like, vinylamide group, and their derivatives. Among these, alkenyl group is preferable.

More specifically, the non-conjugated polymer (2) preferably has a substituent represented by the following formulae (A1) to (A12) as the polymerizable functional group or the substituent having a polymerizable functional group. Among these, the substituents represented by the formulae (A1), (A5), (A8), and (A12) are more preferable, because these substituents can be easily introduced into the iridium complex.

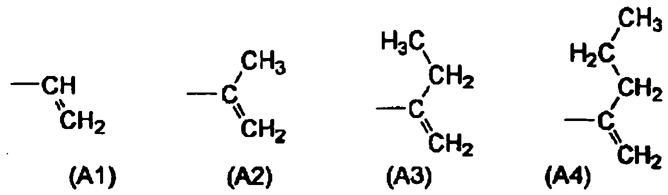

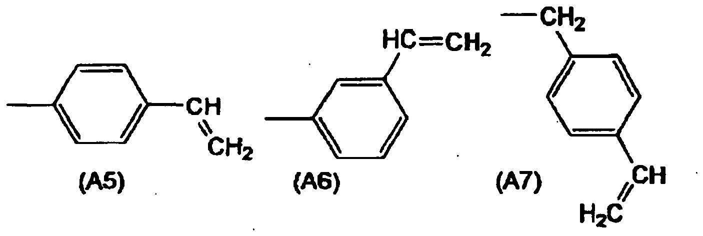

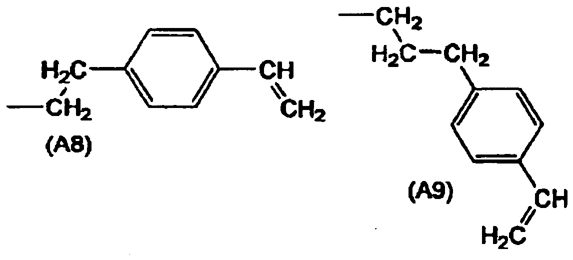

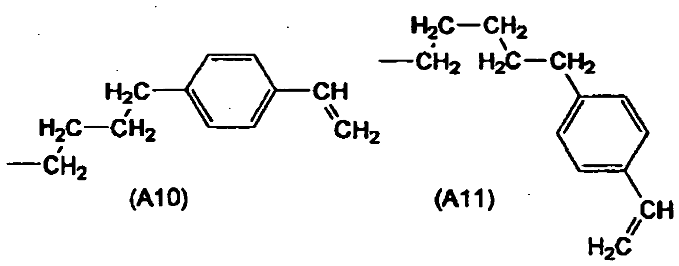

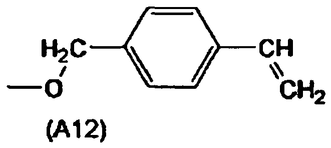

As the compound represented by the formula (2), complexes represented by the following formulae (C13) to (C16) are preferable because an organic EL element having especially extended service life and high luminescence efficiency can be attained, and the complex represented by the formula (C13) is more preferable.

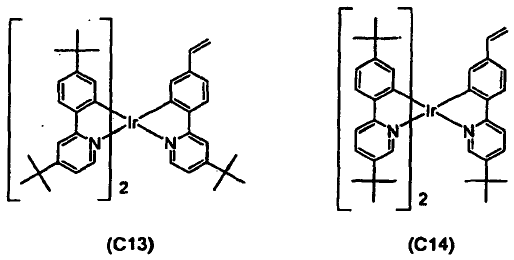

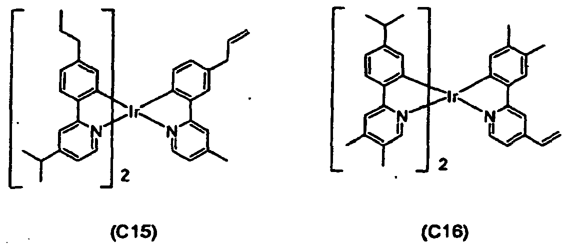

The compound represented by the formula (2) may be used alone or in combination of two or more kinds.

The iridium complex represented by the above formula (2) may be produced by, for example, a method comprising the following steps (i) and (ii):
step (i) : reacting a compound represented by the following formula (2-1) with iridium (III) trichloride trihydrate in a solvent such as 2-ethoxyethanol at about 50 to 150 °C to produce a compound represented by the following formula (2-2); and
step (ii): reacting the compound represented by the following formula (2-2) with a compound represented by the following formula (2-3) in the presence of a base such as potassium carbonate and in glycerin at about 150 to 220°C to produce the iridium complex represented by the above formula (2).

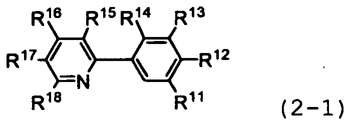

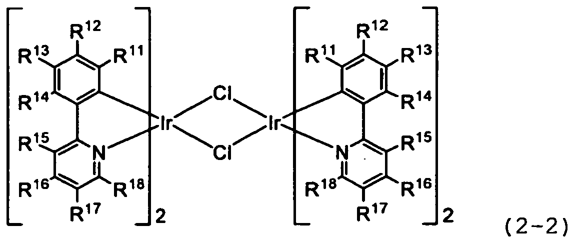

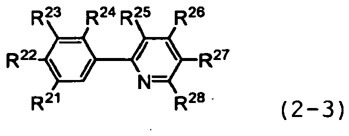

### <Structural unit derived from charge-transporting polymerizable compound>

The polymer having a structural unit derived from the compound represented by the formula (2) is preferably a polymer obtained by copolymerizing one kind or two kinds or more monomers of the iridium complex and a monomer containing at least one kind of polymerizable compound selected from the group consisting of a hole-transporting polymerizable compound and an electron-transporting polymerizable compound. In the present specification, the hole-transporting polymerizable compound and the electron-transporting polymerizable compound are also collectively called a charge-transporting polymerizable compound.

Namely, the non-conjugated polymer (2) is preferably a polymer containing a structural unit derived from one kind or two kinds or more of the iridium complexes, and a structural unit derived from one kind or two kinds or more of the hole-transporting polymerizable compounds or a structural unit derived from one kind or two kinds or more of the electron-transporting polymerizable compounds. Such a non-conjugated polymer (2) provides higher luminescence efficiency, because holes and electrons recombine more efficiently on the structural unit derived from the phosphorescent compound.

Further, the non-conjugated polymer (2) is preferably a polymer containing a structural unit derived from one kind or two kinds or more of the iridium complexes, a structural unit derived from one kind or two kinds or more of the hole-transporting polymerizable compounds, and a structural unit derived from one kind or two kinds or more of the electron-transporting polymerizable compounds. Such high-molecular-weight luminescent material possesses all of the luminescence, hole-transporting, and electron-transporting functions, and thus holes and electrons recombine more efficiently on the iridium complex. As a result, still higher luminescence efficiency is obtained.

There is no particular limitation on the hole-transporting polymerizable compounds and electron-transporting polymerizable compounds as long as they have a polymerizable functional group, and known charge-transporting compounds may be used.

The polymerizable functional group may be any of radical, cation, anion, addition, and condensation-polymerizable functional groups. Among these, a radical polymerizable functional group is preferable because polymers can be easily produced.

The polymerizable functional group is the same as the substituent having a polymerizable functional group containing in the compound represented by the formula (2), and the preferred groups are also the same.

As the hole-transporting polymerizable compounds, specifically, the compounds represented by the following formulae (E1) to (E6) are preferable. The compounds represented by the formulae (E1) to (E3) are more preferable, because these compounds provide a copolymer having large charge mobility therein.

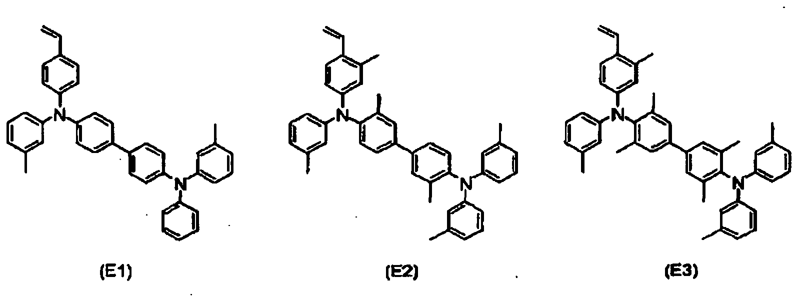

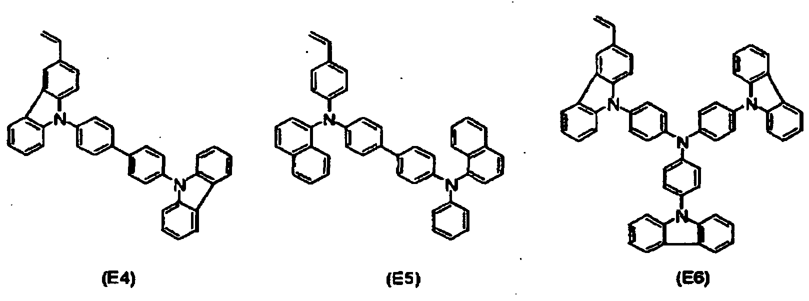

As the electron-transporting polymerizable compounds, specifically, the compounds represented by the following formulae (E7) to (E15) are preferable. The compounds represented by the formulae (E7) and (E12) to (E14) are more preferable, because these compounds provide a copolymer having large charge mobility therein.

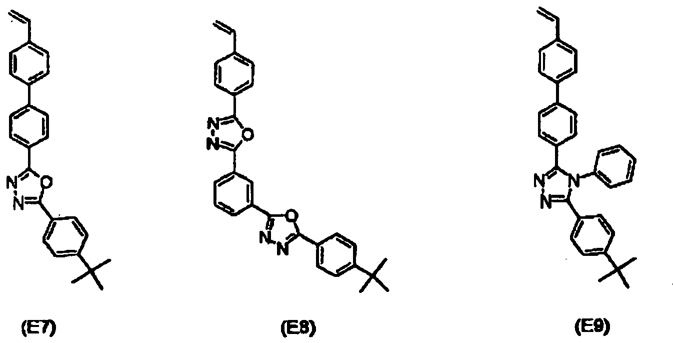

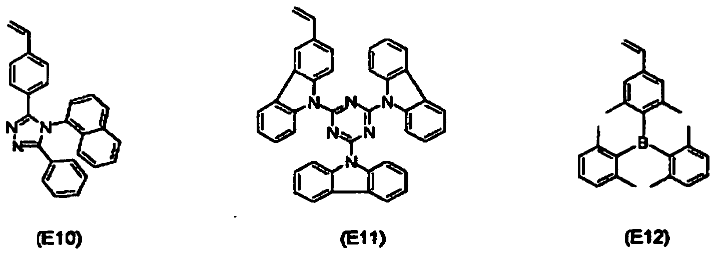

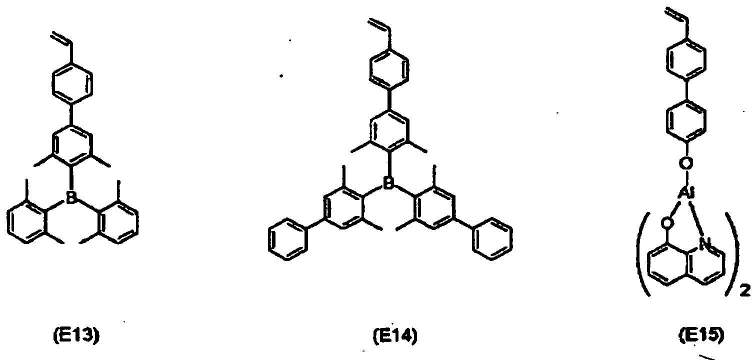

A compound given by replacing the substituent represented by the formula (A1) in the formulae (E1) to (E15) by a substituent represented by any of the formulae (A2) to (A12) may be preferably used. In particular, a compound having a substituent represented by the formulae (A1) or (A5) is preferable, because the functional group can be introduced easily to the polymerizable compounds.

As the non-conjugated polymer (2), more preferable are compounds given by copolymerizing a compound represented by any of the formulae (E1) to (E3) as the hole-transporting polymerizable compound, and a compound represented by any of the formulae (E7) and (E12) to (E14) as the electron-transporting polymerizable compound among these, and the iridium complex, in combination. Thus obtained non-conjugated polymer provides high luminescence efficiency and high maximum attainable luminance, and exhibits excellent durability.

The polymerizable compounds represented by the formulae (E1) to (E15) may be produced by known methods.

The polymer may contain a structural unit derived from the other polymerizable compounds. The other polymerizable compounds are, for example, the compounds having no charge-transporting properties exemplified by alkyl(meth)acrylates such as methyl acrylate and methyl methacrylate, and styrene and its derivatives, but are not limited to them.

The polymer has a weight average molecular weight of preferably from 1,000 to 2,000,000, and more preferably from 5, 000 to 1,000,000. It is preferable that the molecular weight is within the above range, because the polymer is soluble in an organic solvent and uniform thin films are obtained.

A desired polymer mentioned above can be obtained by selecting appropriately the ratio of the iridium complex to the charge-transporting polymerizable compound (hole-transporting and/or electron-transporting polymerizable compound). The polymer may be any of random, block, and alternative copolymers.

In the aforementioned polymer, when the number of the structural unit derived from the iridium complex is "m" and the number of the structural unit derived from the charge-transporting compound (the total number of the structural units derived from the hole-transporting polymerizable compound and/or the electron-transporting polymerizable compound) is "n" (m and n are respectively an integer equal to or larger than 1), the ratio of the structural unit derived from the iridium complex with respect to the whole structural units, that is m/(m + n), is in the range of preferably from 0.001 to 0.5, and more preferably from 0.001 to 0.2. Within this range of m/(m + n), an organic EL element having large charge mobility, a small effect of concentration quenching, and a high luminescence efficiency can be obtained.

Further, in the case where the aforementioned polymer contains the structural unit derived from the hole-transporting compound and the structural unit derived from the electron-transporting compound, the relationship of n = x + y can be satisfied, wherein "n" is as described above, "x" is the number of the structural unit derived from the hole-transporting compound, "y" is the number of the structural unit derived from the electron-transporting compound, and "x" and "y" are respectively an integer equal to or larger than 1. The optimum values of the ratios x/n and y/n are determined by the charge-transporting ability of each structural unit, the charge-transporting properties, concentrations and others of the structural unit derived from the iridium complex, wherein x/n is the ratio of the number of the structural units derived from the hole-transporting compound, and y/n is the ratio of the number of the structural units derived from the electron-transporting compound, with respect to the number of the structural units derived from the charge-transporting compound. When this polymer is used as the only compound for forming the luminescent layer of an organic EL element, the values of x/n and y/n each are in the range of preferably from 0.05 to 0.95, and more preferably from 0.20 to 0.80. Here, the relationship of x/n + y/n = 1 holds.

The polymer may be prepared by any of radical, caution, anion, and addition polymerization methods, but radical polymerization is preferred.

### 3. Organic EL element

### <Organic layer containing luminescent layer>

The organic EL element according to the present invention has one or two or more organic layers sandwiched between an anode and a cathode. At least one of the organic layers contains a phosphorescent compound (A) represented by the formula (1) and a charge-transporting non-conjugated polymer.

FIG. 1 shows an exemplary embodiment for the construction of the organic EL element according to the present invention, but the construction of the organic EL element of the present invention is in no way limited to this exemplary embodiment. In FIG. 1, a luminescent layer 3 is provided between an anode 2, which is provided on a transparent substrate 1, and a cathode 4. The organic EL element may be provided with a hole-injection layer between the anode 2 and the luminescent layer 3, or with an electron-injection layer between the luminescent layer 3 and the cathode 4.

In the above description, an organic layer containing a phosphorescent compound (A) represented by the formula (1) and a charge-transporting non-conjugated polymer can be used as a luminescent layer that has both hole-transporting and electron-transporting properties. As a result, an organic EL element having high luminescence efficiency can be advantageously fabricated without forming a layer of the other organic materials.

There is no particular limitation on the method of producing the organic layer, but the organic layer is, for example, produced as follows. Firstly, a solution obtained by dissolving a phosphorescent compound (A) represented by the formula (1) and a charge-transporting non-conjugated polymer is prepared. There is no particular limitation on the solvent used for preparing the above solution, but there may be used, for example, chlorine-based solvents such as chloroform, methylene chloride, and dichloroethane, ethers such as tetrahydrofuran, and anisole, aromatic hydrocarbons such as toluene, and xylene, ketones such as acetone; and methylethylketone, esters such as ethyl acetate, butyl acetate, and ethylcellosolve acetate, and others. Subsequently, the solution thus prepared is formed into a film on a substrate by a coating method such as ink-jet coating, spin coating, dip coating, and printing. The concentration of the solution is, although it depends on the compound used and film forming conditions, for example, preferably from 0.1 to 10 wt% in the case of spin coating or dip coating. In this way, the organic layer is easily formed, and thereby the production process can be simplified and an element with larger area can be produced.

### <Other materials>

In the forming of each layer described above, a high-molecular-weight material serving as a binder may be admixed. Examples of the high-molecular-weight material include polymethylmethacrylate, polycarbonate, polyester, polysulfone, and polyphenyleneoxide.

Further, the material used for forming each layer described above may be a mixture of materials having different functions, including a luminescent material, a hole-transporting material, and an electron-transporting material, for example. In the organic layer containing the phosphorescent compound and the non-conjugated polymer, the other hole-transporting material and/or electron-transporting material may be admixed in order to support the charge-transporting properties thereof. Such a transporting material may be either a low-molecular-weight compound or a high-molecular-weight compound.

Examples of the hole-transporting material that forms the hole-transporting layer or is admixed in the luminescent layer include a low-molecular-weight triphenylamine derivative such as TPD (N,N'-dimethyl-N,N'-(3-methylphenyl)-1,1'-biphenyl-4,4'-diamine), α-NPD (4,4'-bis[N-(1-naphthyl)-N-phenylamino] biphenyl), and m-MTDATA (4,4',4"-tris(3-methylphenylphenylamino)triphenylamine); polyvinylcarbazole; a high-molecular-weight compound obtained by polymerizing the above triphenylamine derivatives after a polymerizable substituent is introduced into the derivatives, for example, a high-molecular-weight compound having a triphenylamine skeleton disclosed in Japanese Patent Laid-Open Publication No. H8-157575; and a fluorescent high-molecular-weight compound such as polyparaphenylenevinylene and polydialkylfluorene. The hole-transporting material may be used alone or in combination of two or more kinds, or different kinds of the hole-transporting materials may be built-up in layers. The thickness of the hole-transporting layer is not specified in general because it depends on the conductivity and the like of the hole-transporting layer, but it is desirable that the thickness be preferably from 1 nm to 5 µm, more preferably from 5 nm to 1 µm, and particularly preferably from 10 nm to 500 nm.

Examples of the electron-transporting material that forms the electron-transporting layer or is admixed in the luminescent layer include a low-molecular-weight compound such as a quinolinol derivative metal complex (for example, Alq3 (aluminum trisquinolinolate)), an oxadiazole derivative, a triazole derivative, an imidazole derivative, a triazine derivative, and a triarylborane derivative; and a high-molecular-weight compound obtained by polymerizing the above low-molecular-weight compound after a polymerizable substituent is introduced into the low-molecular-weight compound, for example, poly PBD disclosed in Japanese Patent Laid-Open Publication No. H10-1665. The electron-transporting material may be used alone or in combination of two or more kinds, or different kinds of electron-transporting materials may be built-up in layers. The thickness of the electron-transporting layer is not specified in general because it depends on the conductivity and others of the electron-transporting layer, but it is desirable that the thickness be preferably from 1 nm to 5 µm, more preferably from 5 nm to 1 µm, and particularly preferably from 10 nm to 500 nm.

Further, a hole-blocking layer may be disposed adjacent to the luminescent layer on the cathode side thereof so as to suppress holes passing through the luminescent layer and to promote recombination of holes and electrons in the luminescent layer. The hole-blocking layer is formed from a known material such as a triazole derivative, an oxadiazole derivative, and a phenanthroline derivative.

A hole-injection layer may be disposed between the anode and the luminescent layer so as to reduce the hole-injection barrier. The hole-injection layer is formed from a known material such as copper phthalocyanine, a mixture of polyethylene dioxythiophene (PEDOT) and polystyrene sulfonic acid (PSS), and a fluorocarbon.

An insulating layer having a thickness of from 0.1 to 10 nm thick may be disposed between the cathode and the electron-transporting layer, or the cathode and the organic layer that is built up in layers adjacent to the cathode. The insulating layer is formed from a known material such as lithium fluoride, magnesium fluoride, magnesium oxide, and alumina.

As the anode material, there may be used, for example, a known transparent conductive material exemplified by ITO (indium tin oxide), tin oxide, zinc oxide, and a conductive polymer such as polythiophene, polypyrrole, and polyaniline. The surface resistance of the electrode formed from the transparent conductive material is desirably from 1 to 50 Ω/□ (ohm/square). The thickness of the anode is desirably from 50 to 300 nm.

As the cathode material, there may be used, for example, a known cathode material exemplified by an alkali metal such as Li, Na, K, and Cs; an alkaline earth metal such as Mg, Ca, and Ba; Al; a MgAl alloy; and an alloy of Al and an alkali or alkaline earth metal such as AlLi and AlCa. It is desirable that the thickness of the cathode be preferably from 10 nm to 1 µm, and more preferably from 50 to 500 nm. When a highly active metal such as an alkali or alkaline earth metal is used as a cathode, it is desirable that the thickness of the cathode be preferably from 0.1 to 100 nm, and more preferably from 0.5 to 50 nm. Further, in this case, a metal layer that is stable in the air is superposed on the cathode to protect the cathode metal. Examples of the metal that forms the metal layer include Al, Ag, Au, Pt, Cu, Ni, and Cr. It is desirable that the thickness of the metal layer be preferably from 10 nm to 1 µm, and more preferably from 50 to 500 nm.

As the substrate of the organic EL element according to the present invention, an insulating substrate that is transparent to the luminescence wavelength of the above luminescent material is used. The substrate is made of glass, a transparent plastic such as PET (polyethylene terephthalate), polycarbonate, and the like.

The hole-transporting, luminescent, and electron-transporting layers are formed by, for example, resistance heating evaporation, electron beam evaporation, sputtering, ink-jet coating, spin-coating, printing, spraying, and dispensing. Resistance heating evaporation and electron beam evaporation are suitably used in the case of low-molecular-weight compounds. In the case of high-molecular-weight compounds, ink-jet coating, spin-coating, or printing is suitably used.

The anode material is formed into a film by, for example, electron beam evaporation, sputtering, chemical reaction, and coating. The cathode material is formed into a film by, for example, resistance heating evaporation, electron beam evaporation, sputtering, and ion plating.

### 4. Uses

The organic EL element according to the present invention is suitably used as pixels for a matrix or segmented image display device with a known method. In addition, the organic EL element is suitably used also as a plane light source without formed into pixels.

Specifically, the organic EL element according to the present invention is suitably used for applications exemplified by display devices for computers, television sets, personal digital assistances, cellular phones, car navigation systems, video camcorder viewfinders and others, backlights, electrophotography, illumination light sources, recording light sources, exposure light sources, reading light sources, sign boards, display boards, interior accessories, and optical communication.

### Examples

The present invention will be further described in detail with reference to the following Examples.

### Synthesis Example 1

### <Synthesis of iridium complex (C1)>

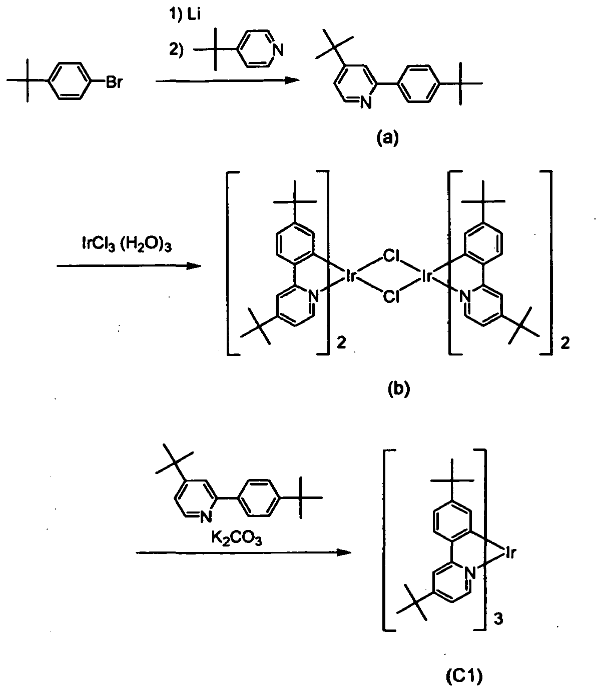

There will be explained with reference to the above reaction scheme. To a mixture of 1.6 g (0.24 mol) of lithium and 200 ml of diethyl ether, 25 g (0.12 mol) of 4-t-butylbromobenzene were added dropwise. After 2 hours of stirring at room temperature, 50 ml of a diethyl ether solution dissolving 16 g (0.12 mol) of 4-t-butylpyridine were further added dropwise, and the resulting reaction solution was stirred at room temperature for 2 hours. Subsequently, oxygen gas was blown into the reaction solution for 30 minutes, and the solution was stirred at room temperature for 12 hours. Water was added to the resulting solution, and then the organic phase was extracted. After the solvent was removed by distillation under reduced pressure, compound (a) was obtained by further distillation under reduced pressure.

Secondly, a mixture of 1.0 g (3.7 mmol) of the compound (a), 0.65 g (1.8 mmol) of iridium trichloride trihydrate, 60 ml of 2-ethoxyethanol and 20 ml of water was heated under reflux for 12 hours. The resulting precipitate was washed with cold methanol, and dried under reduced pressure to obtain compound (b).

Finally, 20 ml of glycerin were added to a mixture of 1.0 g (0.66 mmol) of the compound (b), 0.40 g (1.3 mmol) of the compound (a), and 0.25 g (1.80 mmol) of potassium carbonate, and then the resultant mixture was heated and mixed for 24 hours at 200°C. Water was added to the resulting reaction solution, and then the resulting precipitate was filtered off and purified by silica gel column chromatography to obtain 0.58 g (0.59 mmol) of iridium complex (C1).

Identification results of the compound (C1) are as follows.

### Elemental Analysis

calculated (C₅₇H₇₂IrN₃) : C 69.05, H 7.32, N 4.24; and

measured: C 68.71, H 7.45, N 4.46

Mass Spectroscopy (FAB+): 991 (M⁺)

### Synthesis Examples 2

<Synthesis of iridium complex (C13)>

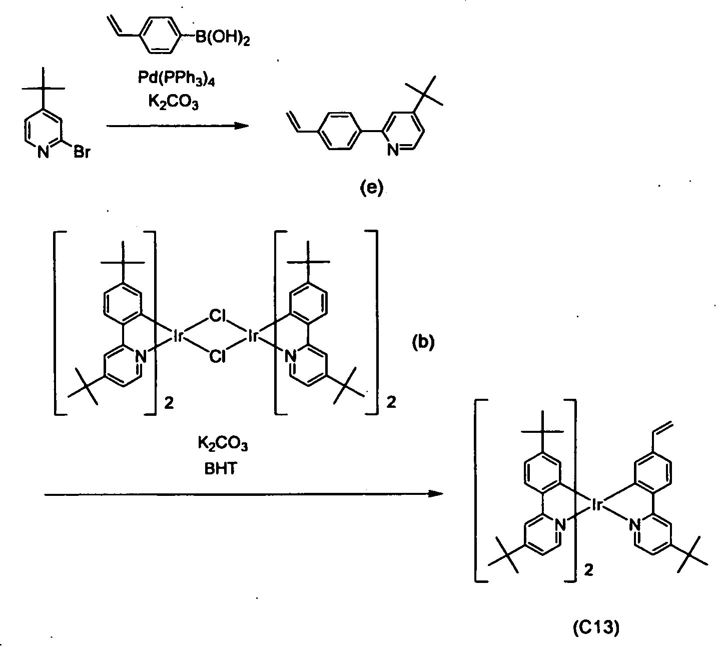

Explanation will be given with reference to the above reaction scheme. To a mixture of 10 g (47 mmol) of 2-bromo-4-t-butylpyridine, 6.9 g (47 mmol) of 4-vinylphenyl boronic acid, 1.0 g (0.87 mmol) of tetrakis (triphenylphosphine) palladium and 17 g (0.12 mol) of potassium carbonate were added 50 ml of 1,2-dimethoxyethane and 25 ml of water, and then the resultant mixture was heated under reflux for 5 hours. An organicphasewas extracted from the resulting reaction solution. After the solvent was removed by distillation under reduced pressure, compound (e) was obtained by purification using silica gel column chromatography.

Secondly, 20 ml of glycerin were added to a mixture of 0.31 g (1.3 mmol) of the compound (e), 1.0 g (0.66 mmol) of the compound (b) prepared as above, 0.22 g (1.6 mmol) of potassium carbonate and 0. 020 g (0.090 mmol) of 2, 6-di-t-butyl-p-cresol, and the resultant mixture was heated and stirred at 200°C for 24 hours. Water was added to the resulting reaction solution and the resulting precipitate was filtered off and purified by silica gel column chromatography to obtain 0.10 g (0.10 mmol) of iridium complex (C13).

Identification results of the compound (C13) are as follows.

### Elemental-Analysis

calculated (C₅₅H₆₆IrN₃): C 68.71, H 6.92, N 4.37; and

measured: C 68.38, H 7.05, N 4.63

Mass Spectroscopy (FAB+): 961 (M⁺)

### Synthesis Example 3

### <Synthesis of copolymer (1)>

In a sealed vessel, 500 mg of the compound represented by the formula (E2) and 500 mg of the compound represented by the formula (E14) were charged, and further dehydrated toluene (9.9 mL) was added. After a toluene solution (0.1 M, 198 µL) of dimethyl-2,2'-azobis(2-methylpropionate) (Trade name: V-601, manufactured by Wako Pure Chemical Industries, Ltd.) was added, freezing and degassing were repeated five times. The vessel was sealed in vacuum and the reaction solution was stirred at 60°C for 60 hours. Subsequently, the reaction solution was added dropwise to 500 mL of acetone to obtain a precipitate. The precipitate was then subjected to reprecipitation-purification twice using a mixed solvent of toluene and acetone, and vacuum-dried overnight at 50°C to obtain copolymer (1).

The copolymer (1) had a weight average molecular weight (Mw) of 118, 500 and a molecular weight distribution index (Mw/Mn) of 2.60. The x/n and y/n values of the copolymer estimated from the results of elemental analysis and ¹³C-NMR measurement were 0.47 and 0.53, respectively.

### Synthesis Example 4

### <Synthesis of copolymer (2)>

Copolymer (2) was synthesized similarly to Synthesis Example 3, except that 500 mg of the compound represented by the formula (E2) and 500 mg of the compound represented by the formula (E14) were replaced by 80 mg of the iridium compound (C13), 460 mg of the compound represented by the formula (E2) and 460 mg of the compound represented by the formula (E14).

The copolymer (2) had a weight average molecular weight (Mw) of 75, 400 and a molecular weight distribution index (Mw/Mn) of 2.36. The m/(m + n) value of the copolymer estimated from the results of an ICP elementary analysis and ¹³C-NMR measurement was 0.040. The x/n and y/n values of the copolymer (2) were 0.50 and 0.50, respectively.

### Comparative Synthesis Example 1

### <Synthesis of copolymer (3)>

Copolymer (3) was synthesized similarly to the synthesis of the copolymer (2), except that 80 mg of the iridium complex (C13) were replaced by 80 mg of the following iridium complex (f).

The copolymer (3) had a weight average molecular weight (Mw) of 59, 900 and a molecular weight distribution index (Mw/Mn) of 2.10. The m/(m + n) value of the copolymer estimated from the results of an ICP elementary analysis and ¹³C-NMR measurement was 0.043. The x/n and y/n values of the copolymer (3) were 0.48 and 0.52, respectively.

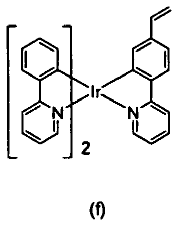

### Example 1

A substrate with ITO (supplied by Nippo Electric Co., Ltd.) was used. The substrate had two ITO (indium tin oxide) electrodes (anode), each having a width of 4 mm, lined on one face of a glass substrate 25 mm square in size.

Firstly, the above substrate with ITO electrodes was treated with UV ozone, and further treated with oxygen plasma for 30 seconds. Still further, the substrate was subjected to the similar plasma treatment for 60 seconds using fluoroform in place of oxygen. Next, 8.0 mg of the iridium complex (C1) and 82 mg of the copolymer (1) were dissolved in 2,910 mg of toluene (Reagent grade, supplied from Wako Pure Chemical Industries, Ltd.). The resulting solution was filtered with a microporous filter having a pore diameter of 0.2 µm to prepare a coating solution. Then, the coating solution was spin-coated on the substrate with ITO electrodes at a rate of 3,000 rpm for 30 seconds. The coating was dried at room temperature (25°C) for 30 minutes to form a luminescent layer. The thickness of the luminescent layer was about 100 nm.

Secondly, the substrate with the luminescent layer was placed in a vacuum deposition chamber. Calcium and aluminum were co-deposited in a weight ratio of 1:10 to form two cathodes, each having a width of 3 mm, lined perpendicularly to the longitudinal direction of the anodes. The thickness of the cathodes was about 50 nm.

Finally, lead wires (wirings) were attached to the anodes and cathodes in argon atmosphere to obtain four pieces of organic EL elements, each 4 mm by 3 mm in size. The resultant organic EL element was allowed to emit light by applying a voltage with a programmable DC voltage/current source (TR6143, supplied by ADVANTEST Corp.). The luminance of the element was measured with a luminance meter (BM-8, manufactured by Topcon Corp.).

Table 1 shows the luminescence color, maximum luminance external quantum efficiency (luminescence efficiency), and the time (life) elapsed until the luminance of the element was degraded by half when the element was subjected to continuous light emission by applying a constant current to the element at and an initial luminance of 100 cd/m², and thereby forced into deterioration.

### Example 2 (not belonging to the scope of the present invention)

An organic EL element was fabricated and evaluated similarly to Example 1, except that 8.0 mg of the iridium complex (C1) and 82 mg of the copolymer (1) were replaced by 90 mg of the copolymer (2). The evaluation results are shown in Table 1.

### Comparative Example 1

An organic EL element was fabricated and evaluated similarly to Example 1, except that 8.0 mg of the iridium complex (C1) were replaced by 8.0 mg of the following iridium complex (g). The evaluation results are shown in Table 1.

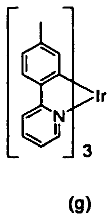

### Comparative Example 2

An organic EL element was fabricated and evaluated similarly to Example 2, except that 90 mg of the copolymer (2) were replaced by 90 mg of the copolymer (3). The evaluation results are shown in Table 1.

**Table 1**

| | Coating material | Luminescence color | Luminescence efficiency (%) | Life (hours) |
|---|---|---|---|---|
| Example 1 | Iridium complex (C1) 8.0 mg Copolymer (1) 82 mg | Green | 6.5 | 5,800 |
| Example 2 * | Copolymer (2) (copolymer containing the structural unit derived from the iridium complex (C13)) | Green | 6.4 | 6,600 |
| Comparative Example 1 | Iridium complex (g) 8.0 mg Copolymer (1) 82 mg | Green | 5.6 | 2,400 |
| Comparative Example 2 | Copolymer (3) | Green | 5.2 | 1,600 |

| | | | | |
|---|---|---|---|---|
| *Reference only | | | | |

## Claims

1. An organic electroluminescence element comprising:
a substrate;
a pair of electrodes formed on the substrate; and
one or plural organic layers formed between the pair of the electrodes, said organic layer containing a luminescent layer,
wherein the luminescent layer contains a phosphorescent compound represented by the following formula (1) and a charge-transporting non-conjugated polymer, 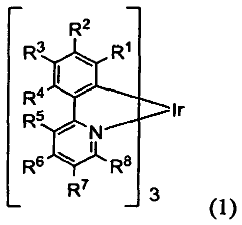
in the formula (1), R¹ to R⁸ each are independently hydrogen atom or an alkyl group having 1 to 20 carbon atoms; a plurality of the alkyl groups may be linked with one another to form a ring;
at least two of R¹ to R⁸ are each the alkyl group; and
at least one of the alkyl groups is an alkyl group having a tertiary or quaternary α-carbon atom.

2. The organic electroluminescence element according to claim 1, wherein all of the alkyl groups are each an alkyl group having a tertiary or quaternary α-carbon atom.

3. The organic electroluminescence element according to claim 2, wherein all of the alkyl groups having a tertiary or quaternary α-carbon atom are each a tertiary butyl group.

4. An application equipped with an organic electroluminescence element according to any one of claims 1 to 3, wherein the application is selected from a display device, a backlight, an electrophotography, an illumination light source, a recording light source, an exposure light source, a reading light source, a sign board, a display board, an interior accessory and an optical communication.

## Patentansprüche

1. Organisches Elektrolumineszenzelement, welches umfasst:
ein Substrat;
ein auf dem Substrat gebildetes Paar Elektroden; und
eine oder mehrere organische Schichten, die zwischen dem Paar Elektroden gebildet ist/sind, wobei die organische Schicht eine lumineszierende Schicht enthält, die eine phosphoreszierende Verbindung der folgenden Formel (1) und ein ladungstransportierendes nicht-konjugiertes Polymer enthält, 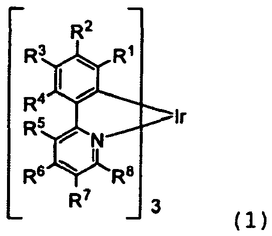
worin in der Formel (1) R¹ bis R⁸ jeweils unabhängig voneinander ein Wasserstoffatom oder eine Alkylgruppe mit 1 bis 20 Kohlenstoffatomen darstellen; eine Vielzahl der Alkylgruppen miteinander unter Bildung eines Rings verknüpft sein können;
mindestens zwei der Gruppen R¹ bis R⁸ jeweils die Alkylgruppe sind; und mindestens eine der Alkylgruppe eine Alkylgruppe mit einem tertiären oder quartären α-Kohlenstoffatom ist.

2. Organisches Elektrolumineszenzelement nach Anspruch 1, wobei alle Alkylgruppen jeweils eine Alkylgruppe mit einem tertiären oder quartären Kohlenstoffatom sind.

3. Organisches Elektrolumineszenzelement nach Anspruch 2, wobei alle Alkylgruppen mit einem tertiären oder quartären α-Kohlenstoffatom jeweils eine tertiäre Butylgruppe sind.

4. Vorrichtung, die mit einem organischen Elektrolumineszenzelement nach einem der Ansprüche 1 bis 3 ausgestattet ist, wobei die Vorrichtung unter einer Anzeigevorrichtung, einem Rücklicht, einer Elektrofotografie, einer Beleuchtungslichtquelle, einer Aufnahmelichtquelle, einer Bestrahlungslichtquelle, einer Leselichtquelle, einer Zeichenwand, einer Anzeigewand, einem inneren Zubehörteil und einer optischen Kommunikation ausgewählt ist.

## Revendications

1. Elément d'électroluminescence organique comprenant :
un substrat ;
une paire d'électrodes formée sur le substrat ; et
une ou plusieurs couches organiques formées entre la paire des électrodes, ladite couche organique contenant une couche luminescente,
où la couche luminescente contient un composé phosphorescent représenté par la formule (1) suivante et un polymère non conjugué transportant des charges, 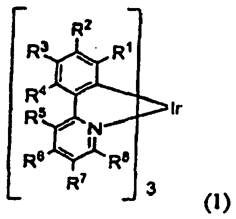
dans la formule (1), R¹ à R⁸ sont chacun indépendamment un atome d'hydrogène ou un groupe alkyle ayant 1 à 20 atomes de carbone ; une pluralité des groupes alkyle peuvent être liés entre eux pour former un cycle ;
au moins deux de R¹ à R⁸ sont chacun le groupe alkyle ; et
au moins l'un des groupes alkyle est un groupe alkyle ayant un atome de carbone α tertiaire ou quaternaire.

2. Elément d'électroluminescence organique selon la revendication 1 où tous les groupes alkyle sont chacun un groupe alkyle ayant un atome de carbone α tertiaire ou quaternaire.

3. Elément d'électroluminescence organique selon la revendication 2 où tous les groupes alkyle ayant un atome de carbone α tertiaire ou quaternaire sont chacun un groupe butyle tertiaire.

4. Application équipée d'un élément d'électroluminescence organique selon l'une quelconque des revendication 1 à 3 où l'application est choisie parmi un dispositif d'affichage, un éclairage arrière, une électrophotographie, une source lumineuse d'illumination, une source lumineuse d'enregistrement, une source lumineuse d'exposition, une source lumineuse de lecture, un panneau de signalisation, un panneau d'affichage, un accessoire intérieur et une communication optique.
